# EUROPEAN PATENT SPECIFICATION

(11) **EP 0 594 843 B1**
(45) Date of publication and mention of the grant of the patent: **03.11.2004**
(21) Application number: 93913922.6
(22) Date of filing: 17.05.1993
(51) Int. Cl.: C07K 16/18, G01N 33/577

(54) **ANTIBODY ASSAY FOR AMYLIN**
ANTIKÖRPER-TESTBESTECK ZUR BESTIMMUNG VON AMYLIN
DISPOSITIF DE DETECTION DE L'AMYLINE UTILISANT DES ANTICORPS

(30) Priority: 15.05.1992 US 883754
(43) Date of publication of application: 04.05.1994
(73) Proprietor: AMYLIN PHARMACEUTICALS, INC., San Diego, CA 92121 (US)
(72) Inventor: PHELPS, Julie, L., San Diego, CA 92126 (US); BLASE, Erich, K., San Diego, CA 92116 (US); KODA, Joy, E., San Diego, CA 92181 (US)
(74) Representative: Duckworth, Timothy John
(86) International application number: PCT/US1993/004651
(87) International publication number: WO 1993/023435

(56) References cited:
- EP-A- 0 289 287
- EP-A- 0 408 294
- WO-A-89/06135
- WO-A-92/11863
- WO-A-92/16845
- SACKS D.B.: 'Amylin-a Glucoregulatory Hormone Involved in the Pathogenesis of Diabetes Mellitus?' CLINICAL CHEMISTRY vol. 42, no. 4, 1996, pages 494 - 495
- NAKAZATO M. ET AL: 'Establishment of radioimmunoassay for human islet amyloid polypeptide and its tissue content and plasma concentration' BIOCHEMICAL AND BIOPHYSICAL RESEARCH COMMUNICATIONS vol. 164, no. 1, 1989, pages 394 - 399
- PERCY A.J. ET AL: 'Development of sensitive immunoassays to detect amylin and amylin-like peptides in unextracted plasma' CLINICAL CHEMISTTRY vol. 42, no. 4, 1996, pages 576 - 585
- ROBERTS A.N. ET AL: 'Molecular and functional characterization of amylin, a peptide associated with type 2 diabetes mellitus' PNAS vol. 86, 1989, pages 9662 - 9666
- PHELPS J.L. ET AL: 'Development and characterization of monoclonal antibodies specific for amylin' HYBRIDOMA vol. 15, no. 5, 1996, pages 379 - 386

## Description

### Background of the Invention

This invention relates to antibody assays for amylin.

Amylin is a protein that is expressed in pancreatic islet beta-cells and secreted into the blood in response to nutrient intake. Its structure and biological activities are described in a publication by Amylin Pharmaceuticals, Inc. (formerly, Amylin Corporation), "Science and Corporate Review", October 1991, which is hereby incorporated by reference herein.

There is a need for monitoring amylin levels in Type 1 diabetics (who have lowered or negligible levels of circulating amylin), in Type 2 diabetics and obese individuals (who have excessive levels of circulating amylin), and in other conditions in which amylin levels may be altered and should be periodically determined or otherwise evaluated. There is also a need for an assay which is specific to amylin (because of its homology to the calcitonin gene-related peptides) and that is sensitive (because of the very small amounts of circulating amylin). There is, furthermore, a need for an assay that can be used to monitor amylin analogs (such as AC-0137) which are being evaluated for use in the treatment of Type 1 diabetes.

EP-A-289287 describes the amino acid sequence of amylin. Also provided are synthetic amylin and amylin-like peptides, a substantially pure amylin, various hexa- and hepta peptides related to amylin and processes for the preparation of diabetes associated peptide including a vector containing a genetic sequence coding for amylin. Antibodies raised against amylin and amylin-like peptides are suggested.

WO-89/06135 describes compounds and methods for blocking the effects of amylin or amylin agonists such as calcitonin gene relate peptide (CGRP) in the treatment of type 2 diabetes. Inhibitors include substituted peptides or sub-peptides of amylin or CGRP, cross-linked amylin and amylin agonists, synthetic amylin, anti-amylin receptor antibodies and anti-idiotype antibodies, and antibodies directed to amylin and amylin agonist active sites.

### Summary of the Invention

Applicant has discovered that antibodies which bind specifically to the amidated C-terminal end of human amylin are particularly advantageous in antibody based assays for detection of human amylin or analogs thereof comprising the amino acid sequence between amino acids 30 and 37 of human amylin and amidated at the C-terminal end. In particular, applicant has discovered that monoclonal antibodies to the C-terminal end of human amylin can be selected which recognize the amino acid sequence between residues 30 and 37 inclusive in amylin and, importantly, can differentiate between the amidated C-terminal end of human amylin and the non-amidated C-terminal end. (Non-amidated human amylin (also referred to as "amylin acid") is virtually, if not completely, inactive while human amylin (the 37th amino acid of which is tyrosine-amide) is fully bioactive. Such antibodies are useful for detecting the presence or amount of an amylin analog in a test sample, and in particular, are useful in a competitive or sandwich assay for amylin.

Thus in a first aspect, the invention features a monoclonal antibody which binds to the amidated C-terminal end of human amylin and not the non-amidated C-terminal end of human amylin.

By "binds or recognizes" is meant that a positive binding result is obtained using standard assay procedures as described below in Example 1.

The term "monoclonal antibody" refers to a purified antibody produced from a single clone of an antibody producing cell. Such monoclonal antibodies are distinct from polyclonal antibodies which include a plurality of antibodies produced by a plurality of antibody-producing cells in a living organism.

In preferred embodiments, the monoclonal antibody recognizes the C-terminal end of human amylin having the amidated amino acid sequence between amino acids 30 and 37 of amylin.

The invention features a monoclonal antibody which binds to the amidated C-terminal end of human amylin, and not to the non-amidated C-terminal end. For example, the invention includes the monoclonal antibody termed F025-27 deposited with the American Type Culture Collection on May 15, 1992 and assigned the number HB 11045.

In another aspect, the invention features an assay for detecting the presence or amount of an amylin analog comprising the amino acid sequence between amino acids 30 and 37 of human amylin and amidated at the C-terminal end. The assay includes use of a monoclonal antibody which binds to the C-terminal end of human amylin. By way of example, an assay using a monoclonal antibody for detecting the presence or amount of an amylin analog may comprise the steps of contacting said amylin analog or a sample suspected of containing said analog with said monoclonal antibody, and determining the presence or amount of said amylin analog, wherein said monoclonal antibody binds to the C-terminal end of human amylin. The assay may be a competitive assay or a sandwich assay. In sandwich format the assay includes a second monoclonal or polyclonal anti-amylin antibody, and the first or second antibody is typically detectably labelled.

By "amylin analog" is meant analogs comprising the amino acid sequence between amino acids 30 and 37 of human amylin which are amidated at the C-terminal end. Such analogs include not only natural occurring forms of amylin (including human and rat, amylin), but also proteins which have an amino acid sequence substantially similar to that of amylin but containing one or more deletions or additions of amino acids within that sequence or including substitution of one or more amino acids generally with a conserved amino acid, for example, glycine may be replaced with a valine, or a positively charged amino acid replaced with other positively charged amino acid. Such amylin analogs have similar or identical biological activity to the naturally occurring amylin most closely related in sequence to that analog. Such analogs are useful in the treatment of various diseases or disease states or conditions and the detection of such analogs is useful to determine that the appropriate level of amylin analog is present within an organism, for example, by removing a test sample from that organism and determining the level of amylin analog within that sample. An amylin analog which may be detected or measured using the inventions described and claimed herein is ^{25,28,29}Pro-h-amylin, also referred to as AC-0137.

In preferred embodiments, the method includes use of a second monoclonal antibody to amylin or a polyclonal antibody to amylin; most preferably the monoclonal antibody which binds to the C-terminal end of human amylin is detectably labeled (e.g., with a radioisotope or a fluorescent or chemiluminescent agent, or with an enzyme); the second monoclonal antibody or polyclonal antibody binds at a location distinct from the C-terminal end of amylin; and the assay is either a competitive antibody assay or a sandwich assay.

Other features and advantages of the invention will be apparent from the following description of the preferred embodiments thereof and from the claims.

### Description of the Preferred Embodiments

The general methodology and steps of antibody assays are described by Greene, U.S. Patent 4,376,110, entitled "Immunometric Assays Using Monoclonal Antibodies"; Antibodies, A. Laboratory Manual, Cold Spring Harbor Laboratory, Chapter 14 (1988); Radioimmunoassay and related methods", A.E. Bolton and W.M. Hunter, Chapter 26 of Handbook of Experimental Immunology, Volume 1, Immunochemistry, edited by D.M. Weir, Blackwell Scientific Publications, 1986; "Enzyme immunoassays: heterogenous and homogeneous systems", R.M. Nakamura, A. Voller, and D.E. Bidwell, Chapter 27 of Handbook of Experimental Immunology, Volume 1, Immunochemistry, edited by D.M. Weir, Blackwell Scientific Publications, 1986; and Current Protocols in Immunology, Chapter 2, Section I, Edited by John E. Coligan, Ada M., Druisbeek, et al., 1991.

Unless historical controls are used, in all such assays (as described below) a control or controls are typically performed, which are designed to give positive and/or negative results, for example, the test may include a known amylin, positive control and a known non-amylin negative control.

Monoclonal and polyclonal antibodies to the amino terminal end of amylin can be produced by standard techniques using either whole amylin or fragments thereof. Antibodies to other regions can similarly be produced. Standard assay formats can be used to assay for amylin analogs. Below are non-limiting examples of the invention. Equivalent procedures will be recognized by those in the art.

Kits including these antibodies are also included in this invention, and include separate containers or vials containing separate labelled or unlabelled antibodies and reagents and controls as discussed above.

### Example 1: Monoclonal Antibody Production to C Terminal End of Amylin

Monoclonal F025-27 which recognizes the C-terminal portion of the amylin peptide (residues 30-37), was produced from an outbred strain of mice, ND4. Mice were immunized with a batch of synthetically produced human amylin that had formed a significant number of intermolecular disulfide cross links producing multimers of the amylin sequence (these links were formed spontaneously over time). This peptide source was administered to the mice in Complete Freund's adjuvant followed by subsequent boosts in Ribi's adjuvant at three week intervals. Each mouse received 30-50 ug of peptide per dose. One out of five mice responded to the immunization protocol with positive serum titers against human amylin, and the spleen from this mouse was fused to the myeloma fusion partner P3/X63-Ag8-6.5.3 by a modification of the method of Oi and Herzenberg, 17 Selected Methods in Cellular Immunology, ed. Barbara Mishell and Stanley Shiigi, 1980, W.H. Freeman and Co. Positive clones were selected by radioimmunoassay using standard techniques. Hybridoma antibody in culture supernatants was immobilized on Dynatech Immulon-2 wells coated with sheep anti-mouse antisera and subsequently blocked with 1% nonfat dry milk. Positive clones were detected by the binding of ¹²⁵I-human amylin labeled by chloramine T iodination. All positive clones were expanded and three clones were frozen. Two out of three clones had similar reactivity with amylin peptide fragments, but only one clone (F025-27) remained stable and was successfully subcloned. It was discovered that this antibody binds to the C-terminal end of amidated human amylin but not non-amidated amylin.

Another method to obtain a clone expressing an antibody to the C-terminal portion of the amylin molecule is to immunize with a C-terminal fragment. The human amylin fragment glycine cysteine glycine and amino acids 28-37 of amylin (termed CGG28-37-NH₂) was conjugated to thyroglobulin using succinimidyl-4-(N-maleimidomethyl) cyclohexane-1-carboxylate (SMCC) and used to immunize ND4, Balb/C and A/J mice. Only A/J mice (5/5) responded with positive serum titers when screened with ¹²⁵I-human amylin labeled by chloramine T iodination although all mice responded to the immunizing fragment. Mice with positive serum titers to intact amylin can be fused and screened as outlined above to yield clones binding to the C-terminal portion of the molecule (residues 28-37).

### Example 2: Sandwich Assay Using Radiolabelled F025-27

Either monoclonal antibodies F002-2.18 or F024-4.4 (which recognize epitopes on amylin distinct from the C-terminal end, and were produced using standard techniques such as described above using human amylin cross-linked to thyroglobulin with glutaraldehyde), or other monoclonals or polyclonal antisera can be used to generate a sandwich assay (IRMA) that quantitates amylin. Dynatech Immulon-2 wells are coated with F002-2.18 or F024-4.4 by overnight incubation of antibody at 20 µg/ml in carbonate buffer, pH9. Wells are subsequently blocked with 1% nonfat dry milk for one hour at room temperature (about 20°C). Sample or standard (50 µl) containing amylin (0-750 pM) is added to the well and incubated for three hours at room temperature. Wells are washed with phosphate buffered saline (PBS), 0.1% Tween-20 detergent, and ¹²⁵I-F025-27 (200,000 cpm/50 µl) is added to each well and incubated an additional three hours at room temperature. Wells are washed with PBS, 0.1% Tween-20 detergent and counted in a Pharmacia LKB gamma counter.

### Example 3: Sandwich Assay Using Enzyme-Labelled F025-27

As in Example 2. monoclonal antibodies F002-2.18 or F024-4.4 or other monoclonal antibodies or polyclonal antisera can be used to generate a sandwich assay (IEMA) that quantitates amylin. Dynatech Immulon-2 wells are coated with F002-2.18 or F024-4.4 by overnight incubation of antibody and 20 µg/ml in carbonate buffer, pH9. Wells are subsequently blocked with 1% nonfat dry milk for one hour at room temperature (about 20°C). Sample or standard (50 µl) containing amylin (0-750 pM) is added to the well and incubated for three hours at room temperature. Cells are washed with phosphate buffered saline (PBS), 0.1% Tween-20 detergent, and F025-27 conjugated to alkaline phosphatase is added to each well and incubated an additional three hours at room temperature. Wells are washed with PBS, 0.1% Tween-20 detergent and enzyme activity is detected using the fluorescent substrate, 4-methyl feryl phosphate and signal is read in a fluorescent plate reader (IDEXX or Dynatech).

### Example 4: Comparison of IRMA and IEMA for Amylin

The Example 2 IRMA and the Example 3 IEMA for human amylin were validated and compared to one another.

The average Minimum Detectable Concentration (MDC) for the amylin IRMA was 2.07 pM. The mean intra-assay variability was 6.7%. The inter-assay variability of 8 clinical samples was 17.8%. Quantitation of amylin controls in the IRMA is shown below:

| Control | Mean |
|---|---|
| Low | 5.4 pM |
| Mid | 32.4 pM |
| High | 110.4 pM |

The average MDC for the amylin IEMA was 1.4 pM. The mean intra-assay variability was 6.3%. The inter-assay variability for the same 8 clinical samples referenced above was 16.2%. Quantitation of amylin controls in the IEMA is shown below:

| Control | Mean |
|---|---|
| Low | 5.8 pM |
| Mid | 28.6 pM |
| High | 107.7 pM |

### Example 5: IRMA and IEMA Sandwich Assays for AC-0137

Assays for AC-0137 were prepared and carried out as described in Example 2 (IRMA) and Example 3 (IEMA), and compared to one another in an assay validation study.

The average MDC for the AC-0137 IRMA was 9.3 pM. The mean intra-assay variability was 7.5%. For clinical samples tested, the inter-assay variability was 16.6%. Quantitation of AC-0137 controls in the IRMA is shown below:

| Control | Mean |
|---|---|
| Low | 14.4 pM |
| Mid | 55.0 pM |
| High | 147.6 pM |

The average MDC for the AC-0137 IEMA was 1.1 pM. The mean intra-assay variability was 3.5% and the inter-assay variability (for clinical samples) was 16.7%. Quantitation of AC-0137 controls in the IEMA is shown below:

| Control | Mean |
|---|---|
| Low | 13.2 pM |
| Mid | 58.8 pM |
| High | 239.3 pM |

### Deposit

Applicant acknowledges its responsibility to replace this culture should it die before the end of the term of a patent issued hereon, 5 years after the last request for a culture, or 30 years, whichever is the longer, and its responsibility to notify the depository of the issuance of such a patent, at which time the deposit will be made irrevocably available to the public.

Other embodiments are within the following claims.

## Claims

1. A monoclonal antibody which binds to the amidated C-terminal end of human amylin and not to the non-amidated C-terminal end of human amylin.

2. A monoclonal antibody according to claim 1 wherein said C-terminal end comprises the amino acid sequence between amino acids 30 and 37 inclusive of human amylin.

3. The monoclonal antibody F025-27 produced by the hybridoma deposited at American Type Culture Collection at Deposit No. HB 11045.

4. An immunoassay for detecting the presence or amount of an amylin analog, said amylin analog comprising the amino acid sequence between amino acids 30 and 37 of human amylin and is amidated at the C-terminal end, which immunoassay comprises the steps of:
contacting said amylin analog or a sample suspected of containing said analog with a monoclonal antibody which binds to the amidated C-terminal end of human amylin and not to the non-amidated C-terminal end of human amylin; and
determining the presence or amount of said amylin analog.

5. An assay according to claim 4 wherein the monoclonal antibody is detectably labelled.

6. An assay according to claim 5 wherein the antibody is radiolabelled or enzyme-labelled.

7. An assay according to any one of claims 4 to 6 wherein an optionally labelled second monoclonal antibody to amylin or an optionally labelled polyclonal antibody to amylin is used in said assay.

8. An assay according to any one of claims 4 to 7 wherein said amylin analog is human amylin.

9. An assay according to any one of claims 4 to 7 wherein said amylin analog is ^{25,28,29}Pro-human amylin.

10. An assay according to any one of claims 4 to 9 wherein said assay is a competitive assay or a sandwich assay.

11. An assay according to any one of claims 4 to 10 wherein the monoclonal antibody is monoclonal antibody F0 25-27 as produced by the hybridoma deposited at American Type Culture Collection at Deposit No. HB 11045.

12. A kit comprising a monoclonal antibody which binds to the C-terminal end of human amylin as defined in any one of claims 1 to 3.

## Patentansprüche

1. Monoklonaler Antikörper, welcher an das amidierte C-terminale Ende von Humanamylin und nicht an das nicht-amidierte C-terminale Ende von Humanamylin bindet.

2. Monoklonaler Antikörper nach Anspruch 1, wobei das C-terminale Ende die Aminosäuresequenz zwischen Aminosäuren 30 und 37 einschließlich von Humanamylin umfasst.

3. Monoklonaler Antikörper P025-27, produziert durch das Hybridom, das hinterlegt ist bei der American Type Culture Collection mit der Zugriffsnummer HB 11045.

4. Immunoassay zum Nachweisen des Vorhandenseins oder der Menge eines Amylin-Analogon, welches Amylin-Analogon die Aminosäuresequenz zwischen Aminosäuren 30 und 37 von Humanamylin umfasst und am C-terminalen Ende amidiert ist, welcher Immunoassay die folgenden Schritte umfasst:
Zusammenbringen des Amylin-Analogon oder einer Probe, die vermutlich das Analogon enthält, mit einem monoklonalen Antikörper, welcher an das amidierte C-terminale Ende von Humanamylin und nicht an das nicht-amidierte C-terminale Ende von Humanamylin bindet; und
Bestimmen der Vorhandenseins oder der Menge des Amylin-Analogons.

5. Assay nach Anspruch 4, wobei der monoklonale Antikörper nachweisbar markiert ist.

6. Assay nach Anspruch 5, wobei der Antikörper radiomarkiert oder Enzym-markiert ist.

7. Assay nach einem der Ansprüche 4 bis 6, wobei ein wahlweise markierter zweiter monoklonaler Antikörper zu Amylin oder ein wahlweise markierter polyklonaler Antikörper zu Amylin bei diesem Assay verwendet wird.

8. Assay nach einem der Ansprüche 4 bis 7, wobei das Amylin-Analogon Humanamylin ist.

9. Assay nach einem der Ansprüche 4 bis 7, wobei das Amylin-Analogon ^{25,28,29}Pro-Humanamylin ist.

10. Assay nach einem der Ansprüche 4 bis 9, wobei der Assay ein kompetitiver Assay oder ein Sandwich-Assay ist.

11. Assay nach einem der Ansprüche 4 bis 10, wobei der monoklonale Antikörper der monoklonale Antikörper FO25-27 ist, wie produziert durch das Hybridom, das hinterlegt ist bei der American Type Culture Collection mit der Zugriffsnummer HB 11045.

12. Kit, umfassend einen monoklonalen Antikörper, welcher an das C-terminale Ende von Humanamylin, wie in einem der Ansprüche 1 bis 3 definiert, bindet.

## Revendications

1. Anticorps monoclonal qui se lie à l'extrémité C-terminale amidée de l'amyline humaine et non à l'extrémité C-terminale non amidée de l'amyline humaine.

2. Anticorps monoclonal selon la revendication 1, dans lequel ladite extrémité C-terminale comprend la séquence d'acides aminés entre les acides aminés 30 et 37 inclus de l'amyline humaine.

3. Anticorps monoclonal F025-27 produit par l'hybridome déposé à l'American Type Culture Collection sous le No. de dépôt HB 11045.

4. Test immunologique pour détecter la présence ou une quantité d'un analogue d'amyline, ledit analogue d'amyline comprenant la séquence d'acides aminés entre les acides aminés 30 et 37 de l'amyline humaine et étant amidé à l'extrémité C-terminale, ce test immunologique comprend les étapes de :
mettre en contact ledit analogue d'amyline ou un échantillon soupçonné de contenir ledit analogue avec un anticorps monoclonal qui se lie à l'extrémité C-terminale amidée de l'amyline humaine et non à l'extrémité C-terminale non amidée de l'amyline humaine ; et déterminer la présence ou une quantité dudit analogue d'amyline.

5. Test selon la revendication 4, dans lequel l'anticorps monoclonal est marqué de façon détectable.

6. Test selon la revendication 5, dans lequel l'anticorps est radiomarqué ou marqué par une enzyme.

7. Test selon l'une quelconque des revendications 4 à 6, dans lequel un second anticorps monoclonal contre l'amyline éventuellement marqué ou un anticorps polyclonal contre l'amyline éventuellement marqué est utilisé dans ledit test.

8. Test selon l'une quelconque des revendications 4 à 7, dans lequel ledit analogue d'amyline est l'amyline humaine.

9. Test selon l'une quelconque des revendications 4 à 7, dans lequel ledit analogue d'amyline est la ^{25,28,29}Pro-amyline humaine.

10. Test selon l'une quelconque des revendications 4 à 9, dans lequel ledit test est un test de compétition ou un test en sandwich.

11. Test selon l'une quelconque des revendications 4 à 10, dans lequel ledit anticorps monoclonal est l'anticorps monoclonal F025-27 produit par l'hybridome déposé à l'American Type Culture Collection sous le No. de dépôt HB 11045.

12. Kit comprenant un anticorps monoclonal qui se lie à l'extrémité C-terminale de l'amyline humaine, tel que défini dans l'une quelconque des revendications 1 à 3.
